# FASCICULE DE BREVET EUROPEEN

(11) **EP 1 502 909 B1**
(45) Date de publication et mention de la délivrance du brevet: **09.03.2011**
(21) Numéro de dépôt: 04291789.8
(22) Date de dépôt: 13.07.2004
(51) Int. Cl.: C07C 59/13

(54) **Utilisation d'un dérivé d'acide (dihydro)jasmonique comme agent desquamant**
Verwendung eines Dihydrojasmonsäurederivates zum Entfernen von Hautschuppen
Use of a dihydrojasmonic acid derivative as a desquamative agent

(30) Priorité: 28.07.2003 FR 0309233
(43) Date de publication de la demande: 02.02.2005
(73) Titulaire: L'Oréal, 75008 Paris (FR)
(72) Inventeur: Neuwels, Michel, 1410 Waterloo (BE); Dalko, Maria, 91190 Gif S/Yvette (FR)
(74) Mandataire: Kromer, Christophe

(56) Documents cités:
- EP-A- 0 585 104
- EP-A- 1 333 021
- EP-A- 1 333 022
- WO-A-93/10756
- DATABASE WPI Section Ch, Week 200204 Thomson Scientific, London, GB; Class D21, AN 2002-029066 XP002215117 & JP 2001 207188 A (POLA CHEM IND INC) 31 July 2001 (2001-07-31)

## Description

La présente invention concerne l'utilisation cosmétique d'un dérivé d'acide (dihydro)jasmonique comme agent desquamant.

Elle se rapporte également à un procédé cosmétique pour atténuer les irrégularités visibles ou tactiles de la surface de la peau, en particulier pour atténuer les rides et ridules et/ou les taches cutanées et/ou lisser la peau, comprenant l'application topique sur la peau d'une composition renfermant, dans un milieu physiologiquement acceptable, au moins un tel dérivé d'acide (dihydro)jasmonique et de la glycérine.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont se différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Une élimination forcée de la couche cornée accélère le renouvellement et permet d'améliorer la qualité de surface de la peau.

On connaît dans l'art antérieur divers agents destinés à accélérer le renouvellement de l'épiderme.

On connaît ainsi les propriétés desquamantes des α-hydroxyacides comme l'acide lactique, l'acide glycolique ou encore l'acide citrique ainsi que des β-hydroxyacides et plus spécialement de l'acide salicylique ainsi que ses dérivés (voir WO-A-93/10756).

Tous ces composés ont une action contre le vieillissement de la peau en favorisant la desquamation, c'est-à-dire l'élimination des cellules mortes situées à la surface de la couche cornée de l'épiderme. Cette propriété "desquamante" est aussi appelée, souvent à tort, propriété kératolytique.

Mais les composés de l'art antérieur peuvent également présenter des effets secondaires, tels que des picotements, des tiraillements, des échauffements et des rougeurs désagréables pour l'utilisateur.

On constate donc que subsiste le besoin d'agents desquamants ayant une action au moins aussi efficace que celle des composés de l'art antérieur, mais ne présentant pas leurs inconvénients.

L'invention a pour but de pallier ces inconvénients de l'art antérieur et de proposer de nouveaux composés, dérivés de l'acide (dihydro)jasmonique, susceptibles de favoriser la desquamation de la peau et/ou de stimuler le renouvellement épidermique, dont l'utilisation n'entraîne pas de picotements, de tiraillements, d'échauffements ou de rougeurs désagréables pour l'utilisateur.

Il est certes, connu de la demande JP2001-199 832 d'utiliser le dihydrojasmonate de méthyle comme desquamant par activation des protéases de la couche cornée, en particulier dans le traitement des peaux sèches. Par ailleurs, il est également fait état dans l'abrégé en anglais du document JP 2001-207188, de l'aptitude du dihydrojasmonate de méthyle à accélérer l'activité péroxydasique de la couche cornée. D'autres dérivés d'acide jasmonique, dans lesquels la fonction oxo est remplacée par une fonction alcool (FR-2 835 525) ou comportant une chaîne latérale insaturée (FR-2 835 526) sont également connus comme agents desquamants.

Toutefois, à la connaissance de la Demanderesse, il n'a encore jamais été suggéré que d'autres composés dérivés d'acide (dihydro)jasmonique pouvaient être utiles comme agents desquamants.

Ces composés sont déjà connus, pour certains, comme agents pro-sébogènes (FR 03/01146, non publiée), en ce sens qu'ils ont pour effet d'augmenter la production de sébum par les sébocytes et de lutter ainsi contre le dessèchement cutané d'origine hormonale qui touche essentiellement les femmes en période péri-ménopausale.

Il n'est toutefois pas mentionné dans ce document que les composés divulgués ont également une fonction desquamante permettant d'envisager leur utilisation dans des compositions cosmétiques destinées à traiter les signes cutanés autres que la peau sèche oligoséborrhéique.

En outre, il n'est pas suggéré de les utiliser dans des compositions renfermant de la glycérine.

La présente invention a donc pour objet l'utilisation cosmétique comme agent desquamant d'un dérivé d'acide (dihydro)jasmonique choisi parmi les composés de formule (I) : 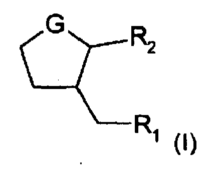 dans laquelle :
G désigne un groupe où ÷Ra désigne ÷un radical hydrocarboné en C1-C₆, linéaire; ou ramifié où R désigne un atome d'hydrogène: et
R₂ est un radical hydrocarboné, linéaire-, ou ramifié non substitué, comportant 5 atomes de carbone, saturé ou insaturé,
et leurs stéréoisomères et sels.

Des exemples de composés préférés pour une utilisation dans la présente invention sont choisis parmi les composés de formule (I) tels que :
- G est un groupe÷ CH-OCH₃ ; R₁ désigne un radical -COOR où R est tel que défini précédemment; et R₂ désigne un radical n-pentyle
ainsi que leurs isomères optiques, sels et complexes cosmétiquement acceptables.

La présente invention a également pour objet un procédé cosmétique pour atténuer les irrégularités visibles ou tactiles de la surface de la peau, en particulier pour atténuer les rides et ridules et/ou les taches cutanées et/ou lisser la peau, comprenant l'application topique sur la peau d'une composition renfermant, dans un milieu physiologiquement acceptable, au moins un dérivé d'acide (dihydro)jasmonique de formule (I), tel que défini précédemment, et de la glycérine.

La composition utilisée selon l'invention est en particulier destinée à être appliquée sur la peau de sujets humains ayant une production de sébum suffisante, c'est-à-dire un taux de sébum au niveau du front supérieur à 100 µg/cm²,

Elle est généralement adaptée à une application topique sur la peau et elle contient donc un milieu physiologiquement acceptable, c'est-à-dire compatible avec la peau et éventuellement avec ses phanères (cils, ongles, cheveux) et/ou les muqueuses.

La quantité de dérivé d'acide (dihydro)jasmonique utilisable selon l'invention est bien entendu fonction de l'effet recherché et peut donc varier dans une large mesure. Pour donner un ordre de grandeur, on peut utiliser le dérivé d'acide (dihydro)jasmonique en une quantité représentant de 0,01% à 20% du poids total de la composition, de préférence en une quantité représentant de 0,1% à 10% du poids total de la composition et, plus préférentiellement, en une quantité représentant de 0,5% à 5% du poids total de la composition.

De son côté, la glycérine peut représenter de 0,1 à 50%, et de préférence de 1 à 10%, du poids total de la composition.

La composition utilisée selon l'invention peut se présenter sous toutes les formes galéniques normalement utilisées dans les domaines cosmétique et dermatologique, et elle peut être notamment sous forme d'une solution éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des dérivés d'acide (dihydro)jasmonique selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 à 30% en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, le palmitate de dextrine et la silice hydrophobe.

L'invention sera maintenant illustrée par les exemples non limitatifs suivants. Dans ces exemples, les quantités sont indiquées en pourcentage pondéral.

### EXEMPLES

### Exemple 1 : Préparation de l'acide (1R,2R) 3-méthoxy-2-[(2Z)-2-pentyl]-cyclopentaneacétique (+/-)

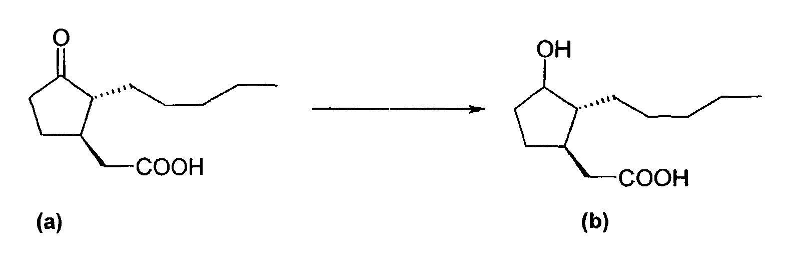

Dans un tricol de 50 ml muni d'un réfrigérant, d'un thermomètre et d'une agitation magnétique, on dissout 1 g (4,8 mmol) d'acide dihydrojasmonique (+/-) (a) dans 15 ml d'éthanol absolu. On ajoute 430 mg (11,4 mmol) de borohydrure de sodium NaBH₄. Le mélange est agité pendant 4 heures à 50°C. Une fois la réaction terminée, on ajoute lentement 5 ml d'eau. Le précipité formé est filtré. Le filtrat est acidifié par de l'acide chlorhydrique jusqu'à pH=5 puis extrait par de l'acétate d'éthyle (3 x 30 ml). La phase organique est séchée sur sulfate de sodium, filtrée sur papier filtre puis concentrée. L'huile obtenue est purifiée par chromatographie sur gel de silice (éluant: dichlorométhane/méthanol). L'huile obtenue est séchée sous vide.

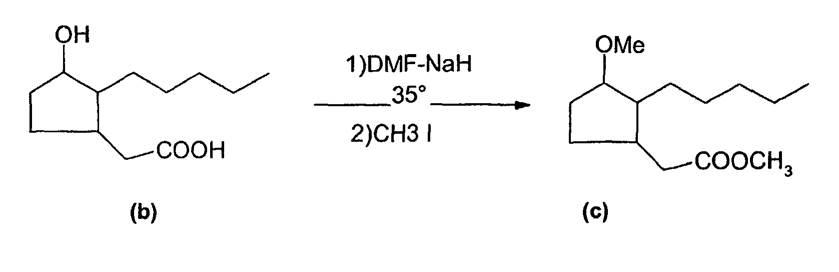

Dans un ballon, on solubilise 2,6 g de composé (b) dans 35 ml de diméthylformamide. On ajoute ensuite 1,2 g d'hydrure de sodium en suspension à 60% dans l'huile, on laisse réagir pendant une heure à 35°C, puis on ajoute 1,8 ml d'iodure de méthyle. On laisse réagir une nuit à 35°C. Après concentration au rotavapeur du milieu réactionnel, le résidu est repris par de l'eau puis extrait par du dichlorométhane. Les phases organiques sont lavées par de l'eau, puis séchées sur sulfate de sodium. Après évaporation à sec, on obtient 3 g d'huile. Ce produit (c) est purifié par chromatographie sur colonne (gel de silice), l'élution étant réalisée par un mélange de pentane / acétate d'éthyle.

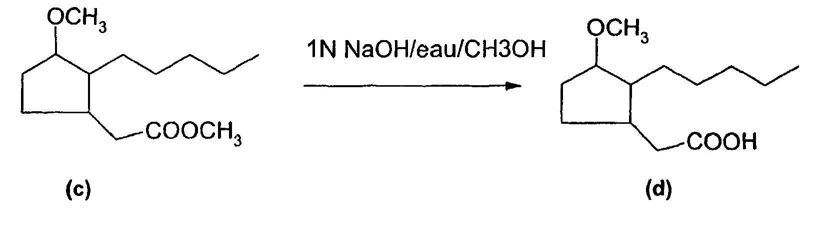

Dans un ballon, on solubilise 0,4 mg de composé (c) dans un mélange de 3 ml de soude 1 N et de 3 ml de méthanol. Après une nuit d'agitation à température ambiante, le méthanol est évaporé. Le reste de la phase aqueuse dilué est lavé à l'acétate d'éthyle. Le pH de la phase aqueuse est ajusté à 2 avec une solution d'acide chlorhydrique 1 N, puis lavé trois fois à l'acétate d'éthyle. Les phases organiques recombinées sont lavées à l'eau, puis séchées sur sulfate de sodium. Après évaporation à sec, on obtient 0,25 g d'huile. Le rendement est de 65%. Les spectres RMN et de masse sont conformes à la structure attendue pour le produit (d).

### Exemple 2 : Mise en évidence des propriétés desquamantes

On a étudié le pouvoir kératolytique du composé préparé à l'Exemple 1 ci-dessus. Ce test a consisté en un comptage des cornéocytes libérés après incubation de lots de stratum corneum, isolé par trypsine/chaleur à partir de plasties de chirurgie, en présence du composé testé.

Des disques de stratum corneum de 4 mm de diamètre ont été découpés à l'emporte pièce et disposés au fond d'une boite de 96 puits. Deux échantillons différents de stratum corneum ont été utilisés.

On a préparé une solution à 1% en poids de composé de l'exemple 1 dans un tampon PBS supplémenté à 0, 1 % en Triton X100. Le pH de la solution a été réajusté à 7,4.

On a ajouté dans chaque puits 50 microlitres de solution à tester ou de solution témoin (tampon PBS supplémenté à 0,1% en TritonX100). L'expérience a été répétée trois fois. On a laissé incuber à 37°C sous agitation pendant 24 heures.

On a ensuite prélevé 10 microlitres de solution, que l'on a disposé en cellule de Mallassez. Les cornéocytes libérés ont été comptés sous microscope.

On a obtenu les résultats suivants, qui expriment la moyenne sur les trois essais du nombre de cornéocytes libérés par microlitre. Les fragments de cornéocytes n'ont pas été comptabilisés.

| | Moyenne | |
|---|---|---|
| | (3 essais par échantillon) | |
| | Echantillon n°1 | Echantillon n°2 |
| Composé 1 | 17±6 | 16±6 |
| Témoin | 6±1 | 6±2 |

Le nombre de cornéocytes libérés après incubation du stratum corneum isolé avec le composé selon l'invention est très supérieur au nombre libéré en présence du tampon seul.

### Exemple 3 : Composition cosmétique

Cette composition est préparée de manière classique pour l'homme du métier. Les quantités sont indiquées en pourcentages pondéraux.

| | |
|---|---|
| Composé de l'Exemple 1 | 0,001 % |
| Méthylparaben | 0,1 % |
| Propylparaben | 0,1 % |
| Lanoline | 5 % |
| Huile de vaseline | 4 % |
| Huile de sésame | 4 % |
| Alcool cétylique | 5 % |
| Monostéarate de glycérol | 2 % |
| Triéthanolamine | 1 % |
| Propylène glycol | 5 % |
| Carbomer 940 | 0,1 % |
| Eau | qsp 100 % |

## Revendications

1. Utilisation cosmétique comme agent desquamant d'un dérivé d'acide (dihydro)jasmonique choisi parmi les composés de formule (I) : 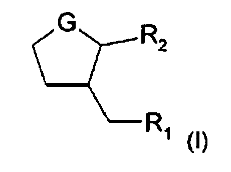 dans laquelle :
G désigne un groupe CH-ORa où Ra désigne un radical hydrocarboné en C₁-C₆, linéaire ou ramifié,
R₁ est un radical -COOR, ou R désigne un atome d'hydrogéne ; et
R₂ est un radical hydrocarboné, linéaire ou ramifié, non substitué, comportant 5 atomes de carbone, saturé ou insaturé,
et leurs stéréoisomères et sels.

2. Utilisation selon la revendication 1, **caractérisée en ce que** le composé de formule (I) est tel que G désigne un groupe -CH-OCH₃; R₁ est un radical -COOH, et R₂ est un radical n-pentyle.

3. Procédé cosmétique pour atténuer les irrégularités visibles ou tactiles de la surface de la peau, en particulier pour atténuer les rides et ridules et/ou les taches cutanées et/ou lisser la peau, comprenant l'application topique sur la peau d'une composition renfermant, dans un milieu physiologiquement acceptable, au moins un dérivé d'acide (dihydro)jasmonique de formule (I), tel que défini dans l'une quelconque des revendications 1 ou 2, et de la glycérine.

4. Procédé selon la revendication 3, **caractérisé en ce que** la composition est destinée à être appliquée sur la peau de sujets humains ayant un taux de sébum au niveau du front supérieur à 100 µg/cm².

## Claims

1. Cosmetic use, as desquamating agent, of a (dihydro)jasmonic acid derivative chosen from the compounds of formula (I): 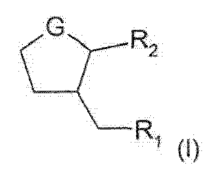 in which:
G denotes a group CH-ORa where Ra is a linear or branched C₁-C₆ hydrocarbon radical,
R₁ is a radical -COOR where R denotes a hydrogen atom; and
R₂ is an unsubstituted, saturated or unsaturated, linear or branched hydrocarbon radical containing 5 carbon atoms,
and their stereoisomers and salts.

2. Use according to Claim 1, **characterized in that** the compound of formula (I) is such that G denotes a group -CH-OCH3; R₁ is a radical -COOH, and R₂ is an n-pentyl radical.

3. Cosmetic method for smoothing the visible or tactile irregularities of the skin surface, in particular for smoothing the wrinkles and fine lines and/or the skin spots and/or smoothing the skin, comprising the typical application, to the skin, of a composition containing, in a physiologically acceptable medium, at least one (dihydro)jasmonic acid derivative of formula (I), as defined in either of Claims 1 and 2, and glycerine.

4. Method according to Claim 3, **characterized in that** the composition is intended to be applied to the skin of human subjects having a sebum level on the forehead greater than 100 µg/cm².

## Patentansprüche

1. Kosmetische Verwendung eines (Dihydro)jasinonsäurederivates, das unter den Verbindungen der Formel (I) ausgewählt sind, als abschuppenden Wirkstoff: 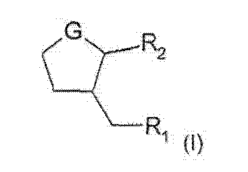 worin bedeuten:
G eine Gruppe CH-ORa, wobei Ra eine lineare oder verzweigte Kohlenwasserstoffgruppe mit 1 bis 6 Kohlenstoffatomen bedeutet,
R₁ eine Gruppe -COOR, wobei R ein Wasserstoffatom bedeutet, und
R₂ eine lineare oder verzweigte, gesättigte oder ungesättigte,
unsubstituierte Kohlenwasserstoffgruppe mit 5 Kohlenstoffatomen.

2. Verwendung nach Anspruch 1, **dadurch gekennzeichnet, dass** die Verbindung der Formel (I) so vorliegt, dass G eine Gruppe -CH-OCH₃ bedeutet; R₁ eine Gruppe -COOH ist; und R₂ eine n-Pentylgruppe bedeutet.

3. Kosmetisches Verfahren zur Milderung sichtbarer oder fühlbarer Unregelmäßigkeiten der Hautoberfläche, insbesondere um Falten und Fältchen und/oder Flecken der Haut abzuschwächen und/oder die Haut zu glätten, das die topische Anwendung einer Zusammensetzung auf die Haut umfasst, die in einem physiologisch akzeptablen Medium mindestens ein (Dihydro)jasmonsäurederivat der Formel (I), wie es in einem der Ansprüche 1 oder 2 definiert ist, und Glycerin enthält.

4. Verfahren nach anspruch 3, **dadurch gekennzeichnet, dass** die Zusammensetzung auf die Haut eines Menschen aufgebracht werden soll, der an der Stirn einen Sebumfluss über 100 µg/cm² aufweist.
